# EUROPÄISCHE PATENTANMELDUNG

(11) **EP 4 671 538 A1**
(43) Veröffentlichungstag der Anmeldung: **31.12.2025**
(21) Anmeldenummer: 25205961.3
(22) Anmeldetag: 09.10.2014
(51) Int. Cl.: F04B 43/02

(54) **VERFAHREN ZUM BETRIEB EINER VERSORGUNGSEINRICHTUNG, DIE EINEN KANAL MIT EINER FLÜSSIGKEIT BEAUFSCHLAGT, SOWIE VERSORGUNGSEINRICHTUNG, HOHLKATHETER UND KATHETERPUMPE**

(30) Priorität: 14.10.2013 EP 13188579
(62) Teilanmeldung aus: 22162535.3
(71) Anmelder: ECP Entwicklungsgesellschaft mbH, 52074 Aachen (DE)
(72) Erfinder: Tiller, Melanie, 52074 Aachen (DE); Er, Sami, 52074 Aachen (DE); Liebing, Reiner, 52074 Aachen (DE)
(74) Vertreter: Pfenning, Meinig & Partner mbB

(57) **Zusammenfassung**

Die vorliegende Erfindung bezieht sich auf eine Versorgungseinrichtung für einen Kanal (8), insbesondere innerhalb eines Hohlkatheters (1), sowie ein Verfahren zum Betrieb einer solchen Versorgungseinrichtung, die einen Kanal (8) mit einer Flüssigkeit beaufschlagt, mit zwei an voneinander beabstandeten Stellen des Kanals angeordneten Pumpen (10, 19), dadurch gekennzeichnet, dass die Parameterwerte wenigstens eines Betriebsparameters beider Pumpen aufeinander abgestimmt gesteuert werden. Durch das Verfahren soll insbesondere bei Verwendung von verschleißfreien Membranpumpen mit einfachen konstruktiven Mitteln ein störungsfreier und genau steuerbarer Betrieb gewährleistet werden.

## Beschreibung

Die Erfindung liegt auf dem Gebiet der Elektrotechnik und der Mechanik und ist mit besonderem Vorteil auf dem Gebiet der Medizintechnik einsetzbar.

Konkret beschäftigt sich die Erfindung mit der Versorgung eines Kanals mit einer Flüssigkeit. Beispielsweise kann es notwendig sein, einen Kanal mit einer Flüssigkeit zu füllen, um die Wände des Kanals zu kühlen oder in dem Kanal angeordnete, bewegte Teile zu kühlen oder zu schmieren und/oder gasfrei zu machen. Zu diesem Zweck ist es grundsätzlich bekannt, einen derartigen Kanal, beispielsweise eine Kanüle, mit einer Kühl- und/oder Schmierflüssigkeit zu versorgen. Die Flüssigkeit kann grundsätzlich mittels einer Pumpe in den oder durch den Kanal bewegt werden.

Oft ist es dabei, insbesondere bei medizinischen Anwendungen, wichtig, dass einerseits durch die Flüssigkeit im Kanal keine dort entstehenden Abriebteile transportiert werden und dass andererseits die Geschwindigkeit, mit der die Flüssigkeit durch den Kanal bewegt wird, möglichst gering aber präzise gesteuert ist. Es kann zudem auch gewünscht sein, dass der Flüssigkeitsverlust aus dem Kanal minimiert wird.

Aus dem Stand der Technik ist beispielsweise aus der DE 20 2005 021 999 U1 ein Wärmetauschsystem mit einer Pumpe bekannt, die den Transport eines Wärmetauschfluids von und zu einem Katheter bewirkt. Es ist ein Strömungsdetektor in Form eines Flügelrades beschrieben, wobei die Drehgeschwindigkeit des durch das Wärmetauschfluid bewegten Flügelrades der Durchflussrate entspricht. Die Geschwindigkeit des Flügelrades wird von außen durch eine Lichtschranke gemessen, die jeweils durch das Passieren einzelner Schaufeln des Flügelrades unterbrochen wird.

Aus der DD 202 805 A1 ist eine Membranpumpe für die medizinische Anwendung bekannt, die dazu dient, Insulin in kleinen Mengen zu fördern. Es ist dort auch ein pulsierender Förderbetrieb beschrieben.

Die DE 694 09 587 T2 offenbart ein Verfahren zum Spülen eines Katheters mit einem Hin- und Rückkanal, um Ablagerungen innerhalb des Katheters möglichst zu minimieren. Es wird dort unter anderem ein pulsierendes Spülen beschrieben, das durch Magnetventile gesteuert werden kann.

Vor dem Hintergrund des Standes der Technik liegt der vorliegenden Erfindung die Aufgabe zugrunde, eine Versorgungseinrichtung zur Beaufschlagung des Kanals mit einer Flüssigkeit sowie ein Verfahren zum Betrieb einer solchen Versorgungseinrichtung zu schaffen, wobei in konstruktiv einfacher Weise eine Lösung angestrebt wird, die in kontrollierter Weise die Steuerung des Durchflusses der Flüssigkeit bei geringer Durchflussrate ermöglicht.

Erfindungsgemäß wird diese Aufgabe durch eine Versorgungseinrichtung und durch ein Verfahren gemäß den unabhängigen Ansprüchen gelöst. Spezielle Ausgestaltungen sind jeweils in den Unteransprüchen beschrieben.

Die Erfindung bezieht sich demgemäß auf ein Verfahren zum Betrieb einer Versorgungseinrichtung, die einen Kanal mit einer Flüssigkeit beaufschlagt, mit einer Membranpumpe. Das Verfahren zeichnet sich dadurch aus, dass die Membranpumpe bezüglich des erzeugten rucks und/oder der Förderrate angesteuert wird.

Erfindungsgemäß wird auch eine Versorgungseinrichtung zur Beaufschlagung eines Kanals mit einer Flüssigkeit mit wenigstens einer Membranpumpe vorgeschlagen. Die Vorrichtung weist eine Steuereinrichtung auf, die die Pumpe bezüglich des erzeugten Drucks (was in einigen Ausführungsformen auch einen Unterdruck oder ein Vakuum umfassen kann) und/oder der Förderrate ansteuert.

Die Erfindung bezieht sich außerdem auf einen Hohlkatheter und/oder eine Katheterpumpe. Wichtig ist hierbei, dass beide über eine erfindungsgemäße Versorgungseinrichtung zur Spülung eines Kanals des Hohlkatheters bzw. der Katheterpumpe in der erfindungsgemäßen Weise verfügen.

Eine Ausführungsform bezieht sich darauf, dass der entsprechend ausgestattete Hohlkatheter innerhalb des Hohlkatheters (vorzugsweise innerhalb des Kanals) eine drehbare Welle aufweist.

Eine weitere Ausführungsform sieht vor, dass die Katheterpumpe, die vorzugsweise einen erfindungsgemäßen Hohlkatheter enthält, ebenfalls von einer drehbaren Welle durchzogen ist. Besonders vorteilhaft hierbei ist, wenn diese drehbare Welle flexibel ist. Für das Beispiel eines Linksherzunterstützungssystems, das intraventrikulär von der Beinarterie eingeführt und bis in den linken Ventrikel des Herzens geschoben wird, heißt das, dass eine solche Pumpe eine drehbare Welle enthält, die außerhalb des Körpers angetrieben ist und einen Rotor im Herzen antreibt; diese Welle muss so flexibel sein, dass sie beispielsweise die Krümmung des Aortenbogens mitvollziehen und hierbei sich trotzdem noch mit hoher Geschwindigkeit drehen kann. Die im zu spülenden Lumen/Kanal drehbare Welle sollte mit einer hohen Drehzahl betreibbar sein, die beispielsweise über 10000 Umdrehungen pro Minute betragen kann.

Die erfindungsgemäße Versorgungseinrichtung gewährleistet hierbei die Versorgung mit Flüssigkeit über die erfindungsgemäße Versorgungseinrichtung, beispielsweise zur Sicherstellung der Luftfreiheit innerhalb des Hohlkatheters bzw. der Katheterpumpe, aber auch zur Schmierung der flexiblen Welle.

Die Erfindung bezieht sich auch auf ein Verfahren zum Betrieb einer Versorgungseinrichtung, die einen Kanal mit einer Flüssigkeit beaufschlagt, mit zwei an voneinander beabstandeten Stellen des Kanals angeordneten Pumpen. Dabei ist es vorgesehen, dass die Parameterwerte wenigstens eines Betriebsparameters beider Pumpen aufeinander abgestimmt gesteuert werden.

Grundsätzlich ist es bekannt und möglich, eine Flüssigkeit mittels einer einzigen Pumpe durch einen Kanal zu bewegen. Durch die Merkmale der Erfindung, insbesondere die Verwendung mehrerer Pumpen, ist jedoch, wenn Betriebsparameter der Pumpen aufeinander abgestimmt werden, beispielsweise die Einstellung eines gemeinsamen Druckniveaus bei Einhaltung von entsprechenden Druckdifferenzen zwischen einem Einlaufbereich und einem Auslaufbereich des Kanals möglich.

Auch die Steuerung einer Durchflussrate ist durch zwei abgestimmte Pumpen derart möglich, dass einerseits eine bestimmte Durchflussrate durch den Kanal eingestellt und andererseits Verlustraten der Flüssigkeit im Einlass- und Auslassbereich oder bei im Verlauf des Kanals vorhandenen Lecks/Öffnungen auf einen bestimmten Wert eingestellt, insbesondere begrenzt werden können.

Um eine besonders gute Einstellbarkeit und damit Steuerbarkeit des Betriebs der Versorgungseinrichtung zu erreichen, bieten sich besonders Membranpumpen zur Realisierung der Versorgungseinrichtung an. Diese können bezüglich des Durchflusses, d. h. der Durchflussrate, besonders genau und reproduzierbar gesteuert werden.

Eine besonders effiziente Steuerung der Versorgungseinrichtung wird möglich, wenn in vorteilhafter Weise an zwei voneinander beabstandeten Stellen in dem Kanal der Flüssigkeitsdruck erfasst wird. Insbesondere kann jede der Stellen, an denen der Flüssigkeitsdruck erfasst wird, einer der Pumpen zugeordnet sein, und es kann mittels der Druckerfassung ein optimales Verhältnis von Saugdruck und Überdruck durch Ansteuerung der Pumpen realisiert werden. Eine solche Steuerung ist insbesondere dann wichtig, wenn der Kanal nicht geschlossen ringförmig ausgebildet ist, sondern einen Ansaugbereich aufweist, in dem dem Kanal von außen aus einem Flüssigkeitsreservoir Flüssigkeit zugeführt wird, und/oder einen Auslaufkanal aufweist, durch den Flüssigkeit aus dem Kanal in ein Auffangreservoir abgeleitet wird.

Die entsprechenden Drucksensoren können separat im Kanal aufgebaut sein, sie können jedoch auch jeweils in eine der Pumpen mit integriert sein.

Als besonders vorteilhaft hat sich bei dem erfindungsgemäßen Verfahren herausgestellt, dass die abgestimmten Parameterwerte der beiden Pumpen zeitlich nach einem festen Schema veränderlich sind und insbesondere nach einer Anlaufphase zeitlich periodisch verändert werden. In der Anlaufphase kann beispielsweise wenigstens eine der Pumpen langsam in ihrer Leistung gesteigert werden. Es kann jedoch auch eine Leistungsspitze angestrebt werden, so dass die Flüssigkeit zunächst mit einer hohen Durchflussrate durch den Kanal strömt, wobei die Durchflussrate nach der Anfangsphase wieder absinkt.

Unabhängig von der Gestaltung der Anlaufphase kann vorgesehen sein, dass der Druck wenigstens einer der Pumpen periodisch steigend und fallend gesteuert wird oder dass entsprechend eine periodisch steigende und fallende Durchflussrate eingestellt wird. Dies ist insbesondere dann vorteilhaft, wenn innerhalb des Kanals bewegte Teile vorhanden sind, wie beispielsweise innerhalb einer Kanüle eine antreibbare Welle, die ihrerseits durch Abrieb kleine Partikel freisetzt. Üblicherweise sollen diese Partikel nicht entlang des Kanals weiterbewegt werden, jedoch soll dennoch die Flüssigkeit transportiert werden. Ein Variieren der Betriebsparameter der Pumpen erlaubt ein effizientes Spülen des Kanals mit der Flüssigkeit, wobei durch die nichtstationäre Strömung alle Teile des Kanals erreicht werden. Durch die Phasen geringeren Durchsatzes der Flüssigkeit können die Partikel in der Strömung zur Ruhe kommen, so dass der Transport der Partikel entlang des Kanals minimiert werden kann.

Ein Variieren der Parameter der Pumpen kann außer der Variation der Leistung jeder einzelnen der Pumpen auch beispielsweise ein Variieren der Leistungsdifferenz oder einer durch die Pumpen erzeugten Druckdifferenz sein. Die Druckdifferenz sorgt für die Beschleunigung der Flüssigkeit und somit eine periodisch schwankende Druckdifferenz für einen entsprechend periodisch schwankenden Flüssigkeitstransport.

Nachfolgend wird unter einer Flüssigkeit eine zum Spülen der Welle verwendete Flüssigkeit verstanden. In einigen Ausführungsbeispielen ist dies nicht eine von der Pumpe zu fördernde Flüssigkeit, obgleich Spuren order geringe Menge der durch die Pumpe zu fördernden Flüssigkeit auch in den Kanal gelangen kann. In anderen Ausführungsbeispielen ist die von der Pumpe zu fördernde Flüssigkeit diejenige welche zur Spülung verwendet werden kann.

Eine weitere vorteilhafte Ausgestaltung der Erfindung sieht vor, dass die abgestimmten Parameterwerte der beiden Pumpen in einem Verhältnis zueinander stehen, das von erfassten Werten des Flüssigkeitsdrucks im Kanal in vorbestimmter Weise abhängig ist. Auf diese Weise kann der Flüssigkeitsdruck im Kanal bzw. eine Flüssigkeitsdruckdifferenz periodisch gesteuert werden.

Die Förderleistungen der Pumpen können anhand verschiedener Messgrößen bestimmt werden. In einigen Ausführungsbeispielen sind die Messgrößen bzw. Betriebsparameter zur Bestimmung der Förderleistung die Hubfrequenz der Membran, und/oder die Hubhöhe der Membran und/oder die Auslenkung der Membran. Dabei kann zur Bestimmung der Förderleistung auf eine der obigen Messgrößen oder eine Kombination mindestens zweier der obigen Messgrößen zurückgegriffen werden. Eine weitere Möglichkeit zur Bestimmung der Förderleistung ist in einigen Ausführungsbeispielen die elektrische Leistungsaufnahme der Pumpe, insbesondere unter Berücksichtigung des herrschenden Flüssigkeitsdrucks.

Die abgestimmten Betriebsparameter beider Pumpen können somit z. B. die jeweiligen Förderleistungen sein. Es kann dann beispielsweise auch eine bestimmte Differenz der Förderleistungen zwischen den beiden Pumpen eingestellt werden. Dies kann dann beispielsweise bedingen, dass eine bestimmte Verlustrate im Verlauf des Kanals für den Flüssigkeitstransport erzeugt wird.

Eine weitere vorteilhafte Ausgestaltung der Erfindung sieht vor, dass die abgestimmten Betriebsparameter beider Pumpen die jeweiligen Werte des durch die Pumpen erzeugten Flüssigkeitsdrucks sind. Der Flüssigkeitsdruck lässt sich in dem Kanal besonders einfach und genau erfassen, so dass durch eine Steuerung der Pumpen beispielsweise ein bestimmter Quotient der Druckwerte oder eine bestimmte Differenz der Druckwerte einstellbar ist. Der Quotient und/oder die Differenz können auch periodisch veränderlich eingestellt werden, um eine stationäre Strömung mit Totwassergebieten zu vermeiden.

Eine weitere vorteilhafte Ausgestaltung der Erfindung sieht vor, dass die abgestimmten Betriebsparameter beider Pumpen die jeweiligen elektrischen Leistungsaufnahmen der Pumpen sind. Dazu kann jeder Pumpe ein elektrischer Sensor zur Erfassung der Leistungsaufnahme der Pumpe, insbesondere zur Erfassung der Stromaufnahme, zugeordnet sein.

Es kann zudem vorteilhaft vorgesehen sein, dass die abgestimmten Betriebsparameter beider Pumpen die jeweiligen Durchflussraten der Pumpen sind. Die Durchflussraten können beispielsweise durch Durchflussmesssensoren separat erfasst werden oder auch durch die Aufnahme der Betriebsparameter der Pumpen, beispielsweise die Leistungsaufnahme und den herrschenden Fluiddruck.

Zudem kann vorteilhaft vorgesehen sein, dass zwischen den beiden Pumpen eine feste Druckdifferenz und/oder eine feste Differenz der Durchflussrate eingestellt wird. Dabei kann insbesondere vorgesehen sein, dass die Differenz der Durchflussraten beider Pumpen kleiner ist als 100 Milliliter pro Tag, insbesondere kleiner ist als 10 Milliliter pro Tag oder kleiner als 1 Milliliter pro Tag.

Entsprechende Verlustraten werden an den Öffnungen des Kanals eingestellt. Beispielsweise kann der Kanal einen Transportkanal und einen Rückflusskanal aufweisen, wobei der Transportkanal beispielsweise an einer am Ende einer Kanüle angeordneten Blutpumpe endet und der Rückflusskanal an derselben Stelle beginnt. Ein Teil der Flüssigkeit, der die Differenz der Durchflussraten ausmacht, kann dann beispielsweise durch die Blutpumpe abströmen, diese spülen und im implantierten Zustand in den Körper eines Patienten abgeleitet werden. In einer Weiterbildung der Erfindung wird in einem solchen Anwendungsfall als Flüssigkeit eine biokompatible, gesundheitsverträgliche Flüssigkeit gewählt, wie beispielsweise eine Kochsalzlösung.

Bei einer vorteilhaften Betriebsform des Verfahrens kann außerdem vorgesehen sein, dass die Bewegungsrichtung der Flüssigkeit umgekehrt wird. Eine solche Umkehrung der Bewegungsrichtung der Flüssigkeit kann periodisch vorgesehen sein oder auch nur zu bestimmten Anlässen. Üblicherweise wird bei der Verwendung zu einer Spülung eines Pumpenkatheters eine Transportrichtung der Flüssigkeit von einem proximalen Ende des Katheters zu einem distalen Ende des Katheters und durch einen Rückflusskanal zurück in einen Auffangbehälter gewählt.

Die Erfindung bezieht sich außer auf ein Verfahren zum Betrieb einer Versorgungseinrichtung auch auf die Gestaltung einer Versorgungseinrichtung zur Beaufschlagung eines Kanals mit einer Flüssigkeit, mit wenigstens zwei Pumpen, insbesondere Membranpumpen, die an voneinander beabstandeten Stellen des Kanals angeordnet sind, sowie mit einer Steuereinrichtung, die die Pumpen bezüglich des erzeugten Drucks und/oder der Förderrate einzeln ansteuert.

Die Steuereinrichtung muss derart gestaltet sein, dass sie eine abgestimmte Ansteuerung der einzelnen Pumpen erlaubt. Sie kann einer der Pumpen zugeordnet oder auch als separate zentrale Steuereinheit ausgebildet sein. Die Steuereinrichtung kann auch zur Regelung von Betriebsparametern der Pumpen dienen und ist dann mit Sensoren zur Erfassung von Messwerten verbunden.

Beispielsweise kann vorgesehen sein, dass jeder Pumpe ein Flüssigkeitsdrucksensor zugeordnet ist. Durch die Steuereinrichtung kann dann ein bestimmtes Druckverhältnis zwischen Saugdruck und Überdruck oder ein bestimmter Quotient der durch die beiden Pumpen erzeugten Drücke oder eine bestimmte Druckdifferenz ausgesteuert werden.

Da die Messgrößen zur Bestimmung der Förderleistung der Pumpen druckabhängig sein können, können die Pumpen beispielsweise als Drucksensoren betrieben werden, wenn ihre Leistungsaufnahme erfasst und der Steuereinrichtung zugeführt wird. Jedoch kann die Leistungsaufnahme auch ein Indikator für die mittels der jeweiligen Pumpe erzielte Durchflussrate sein und als solche erfasst werden. Hierzu ist üblicherweise zudem der herrschende Flüssigkeitsdruck zu berücksichtigen, so dass bei einem solchen Betrieb der gleichzeitige Betrieb von Druckmesssensoren vorteilhaft ist.

Es können jedoch Durchflussratensensoren vorgesehen sein, von denen jeweils einer jeder der Pumpen zugeordnet ist. Es kann dann durch die Steuereinrichtung ein bestimmtes Verhältnis der Durchflussraten im Bereich der ersten und der zweiten Pumpe oder eine vorbestimmte Differenz eingestellt werden. Eine solche Differenz der Durchflussraten kann beispielsweise auch periodisch veränderlich angesteuert werden.

Im Folgenden wird die Erfindung anhand von Ausführungsbeispielen in Figuren einer Zeichnung gezeigt und anschließend beschrieben. Dabei zeigt
- Fig. 1: einen Hohlkatheter mit einer antreibbaren Welle sowie einer erfindungsgemäßen Versorgungseinrichtung,
- Fig. 2: das Ende eines Hohlkatheters in einem Längsschnitt mit einer distal befestigten Rotationspumpe für den Betrieb in einem Blutgefäß,
- Fig. 3: einen Querschnitt durch einen Hohlkatheter,
- Fig. 4: einen Querschnitt durch einen weiteren Hohlkatheter,
- Fig. 5: einen Verfahrensablauf für ein Verfahren zum Betrieb einer Versorgungseinrichtung,
- Fig. 6: ein Diagramm, das den zeitlichen Verlauf von Durchflussraten in drei verschiedenen Varianten wiedergibt, sowie
- Fig. 7: ein Diagramm, das einen zeitlichen Verlauf des Flüssigkeitsdrucks wiedergibt.

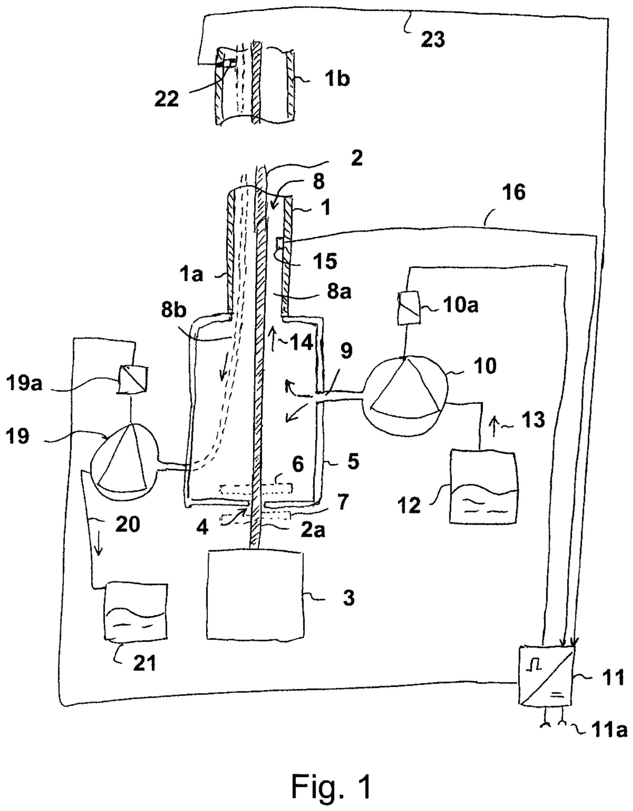

Figur 1 zeigt einen Hohlkatheter 1 in einer in Längsrichtung unterbrochenen Darstellung, wobei ein im medizinischen Gebrauch proximales Ende 1a im unteren Bereich und ein distales Ende 1b im oberen Bereich dargestellt ist. Am distalen Ende des Hohlkatheters 1 kann beispielsweise eine implantierbare Blutpumpe, speziell für den Betrieb in einem Blutgefäß und/oder einer Herzkammer, vorgesehen sein.

Innerhalb des Hohlkatheters 1 verläuft eine drehbar antreibbare Welle 2. Diese dient beispielsweise zum Antrieb einer Blutpumpe und ist an ihrem proximalen Ende 2a mit einem Antriebsmotor 3 verbunden. Die Welle 2 kann im Bereich einer Durchführung 4 in ein Koppelgehäuse 5 eingeführt sein, wobei die Durchführung 4 derart gestaltet ist, dass durch eine Dichtung das Passieren eines Mediums entlang der Welle in das Koppelgehäuse 5 hinein oder aus dem Koppelgehäuse 5 heraus verhindert wird.

Es ist jedoch auch die Lösung denkbar, dass die rotierende Antriebsbewegung mittels einer Magnetkupplung durch eine geschlossene Wand des Koppelgehäuses 5 übertragen wird, indem ein erstes Magnetelement 6 innerhalb des Koppelgehäuses magnetisch mit einem zweiten Magnetelement 7 gekoppelt ist, das außerhalb des Koppelgehäuses 5 auf einem mit dem Motor 3 verbundenen Wellenstumpf befestigt ist. Die Welle 2 weist dann zwischen dem Motor 3 und ihrem weiteren Verlauf im Koppelgehäuse 5 eine Unterbrechung auf, und die entsprechende Wand des Koppelgehäuses 5 ist durchgehend und ohne eine Öffnung ausgebildet. Die Magnetelemente 6, 7 sind in Figur 1 als Alternative gestrichelt dargestellt.

Die Antriebswelle 2 ist beispielsweise aus Litzen, insbesondere in verdrehter oder verseilter Form, hergestellt oder als Schraubenfeder ausgebildet oder in einer Kombination beider Varianten durch eine Seele mit einer diese umgebenden Schraubenfeder gebildet, um einerseits hohe Umdrehungszahlen im Bereich von einigen tausend Umdrehungen pro Minute übertragen zu können und dabei andererseits biegsam zu sein.

Um eine solche Welle einerseits im Betrieb zu kühlen und andererseits die Reibung durch Schmierung zu vermindern, ist üblicherweise innerhalb des in dem Hohlkatheter 1 gebildeten Kanals 8 eine Kühl- und Schmierflüssigkeit vorgesehen, die vorteilhaft biokompatibel ist. Die Flüssigkeit wird dem Koppelgehäuse 5 über einen Zuflusskanal 9 zugeführt und entlang des Kanals 8 transportiert. Hierzu ist der Zuflusskanal 9 mit einer ersten Pumpe, die in dem Ausführungsbeispiel als Membranpumpe 10 ausgebildet ist, verbunden. Membranpumpen haben in diesem Zusammenhang die Eigenschaft, sehr zuverlässig und reproduzierbar ansteuerbar zu sein, um erzeugte Drücke und Durchflussraten exakt steuern zu können. Als besonders vorteilhaft erweist sich in diesem Zusammenhang die Verwendung von magnetisch angesteuerten Membranpumpen. Deshalb ist in Figur 1 eine Magneteinrichtung 10a dargestellt, die als Antrieb der Membranpumpe 10 dient, wobei die Magneteinrichtung 10a durch eine elektrische Steuereinrichtung 11 angesteuert ist.

Die Membranpumpe 10 saugt aus einem Zulaufreservoir 12 Flüssigkeit an, wie durch den Pfeil 13 dargestellt ist, und transportiert diese mit einer einstellbaren Durchflussrate und einem einstellbaren Druck über den Zuflusskanal 9 in das Koppelgehäuse 5 hinein. In dem Koppelgehäuse 5 verteilt sich die Flüssigkeit und bewegt sich insbesondere in Richtung des Pfeils 14 entlang des Kanals 8 in Richtung des distalen Endes 1b des Hohlkatheters. Die Bewegung entlang des Kanals 8 kann beispielsweise durch die Drehung der Welle 2 unterstützt werden, wenn diese eine wenigstens teilweise helixförmige Außenkontur aufweist und in einer geeigneten Rotationsrichtung rotiert.

Obgleich die Drehung der Welle 2 die Bewegung der Flüssigkeit entlang des Kanals 8 unterstützen kann, ist es in einigen Ausführungsbeispielen möglich, den Beitrag der Drehung der Welle zur Förderleistung zu bestimmen, um so die Förderleistung der Pumpe(n) anzupassen. D.h. die aufgrund der Drehung der Welle auftretende Förderleistung wird durch eine Anpassung der Förderleistung der Pumpen kompensiert. Die Bestimmung der Förderleistung aufgrund der Drehung der Welle kann dann auch als Störgröße interpretiert werden, welche durch die Anpassung der Förderleistung der Pumpen ausgeglichen wird, um eine vorbestimme Förderleistung durch den Kanal zu gewährleisten. Die Förderleistung der Welle 8 kann unter anderem von der Drehzahl der Welle, eventuellem Verschleiß an der Welle, der Biegung des Katheters oder ähnlichem abhängen. Obgleich diese Größen bestimmbar sind, ist eine Kompensation der resultierenden Förderleistung der Welle durch die Pumpe oftmals einfacher.

Üblicherweise können mittels der Ansteuerung der Membranpumpe 10 Durchflussraten im Bereich von Mikrolitern oder Millilitern pro Stunde eingestellt werden.

Um entsprechende Durchflussraten und/oder Drücke geeignet steuern oder regeln zu können, ist wenigstens ein geeigneter Sensor 15 in dem Kanal 8 vorgesehen, der mittels einer Kommunikationsleitung 16 mit der Steuereinrichtung 11 verbunden ist. Der Sensor 15 kann beispielsweise als Drucksensor, als Durchflussratensensor oder als kombinierter Sensor zur Erfassung des Drucks und der Durchflussrate ausgebildet sein.

In dem gezeigten Ausführungsbeispiel ist der Sensor 15 der ersten Membranpumpe 10 zugeordnet und erfasst den durch diese erste Pumpe erzeugten Druck und/oder die entsprechende Durchflussrate.

Der Kanal 8 ist gemäß dem Ausführungsbeispiel der Figur 1 in Längsrichtung unterteilt in einen ersten Kanalbereich 8a, der in Richtung von dem Koppelgehäuse 5 zum distalen Ende 1b des Hohlkatheters 1 in Richtung des Pfeils 14 durchströmt wird, und einen zweiten Kanalbereich 8b, der als Rückkanal ausgebildet ist. Die beiden Kanalbereiche 8a, 8b sind somit in Reihe geschaltet und bilden insgesamt den Kanal 8.

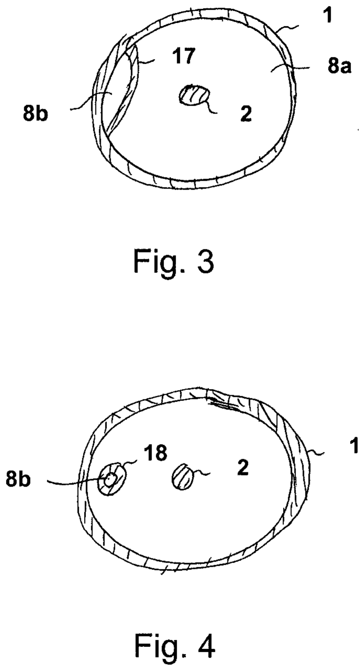

Der Rückkanal 8b kann beispielsweise durch eine Trennwand 17, die in Figur 3 dargestellt ist, von dem ersten Kanalbereich 8a getrennt sein, oder der zweite Kanalbereich/Rückkanal 8b kann durch eine Kanüle 18 gebildet sein, die innerhalb des Hohlkatheters 1 verläuft. Diese Variante ist in Figur 4 im Querschnitt dargestellt.

Der Rückkanal 8b ist gemäß Figur 1 derart ausgebildet, dass er einen Rückfluss der Flüssigkeit in das Koppelgehäuse 5 und von dort in eine zweite Membranpumpe 19 bewirkt. Auch die zweite Membranpumpe 19 kann vorteilhaft als magnetische Membranpumpe mit einer Magneteinrichtung 19a ausgebildet sein, die durch die Steuereinrichtung 11 angesteuert ist und den Antrieb der Membranpumpe 19 bildet. Die Membranpumpe 19 saugt aus dem Rückkanal 8b die Flüssigkeit ab und leitet diese über einen Ablaufkanal 20 in ein Ablaufreservoir 21.

Die Steuereinrichtung 11 ist zudem mit einem zweiten Sensor 22 verbunden, der, ebenso wie der erste Sensor 15, als Durchlaufsensor und/oder als Drucksensor ausgebildet sein kann und der dem Rückkanal 8b und damit der zweiten Membranpumpe 19 zugeordnet ist. Beispielsweise kann durch den zweiten Sensor 22 die Durchflussrate des Rückkanals 8b oder der Saugdruck der zweiten Membranpumpe 19 erfasst werden. Die durch den zweiten Sensor 22 erfassten Parameter werden über eine zweite Kommunikationsleitung 23 der Steuereinrichtung 11 zugeführt.

Die Steuereinrichtung 11 ist ihrerseits mit einem elektrischen Versorgungsanschluss 11a verbunden, der die Steuereinrichtung mit einer niedrigen Gleichspannung (Kleinspannung) versorgt. Die Steuereinrichtung 11 erzeugt Impulse, die den Magneteinrichtungen 10a, 19a zum Antrieb der ersten und zweiten Membranpumpe 10, 19 zugeleitet werden. Mittels der Frequenz und des Hubs der durch die Steuereinrichtung 11 erzeugten Impulse können die Durchflussraten und/oder Drücke gesteuert werden, die durch die erste und zweite Membranpumpe 10, 19 erzeugt werden.

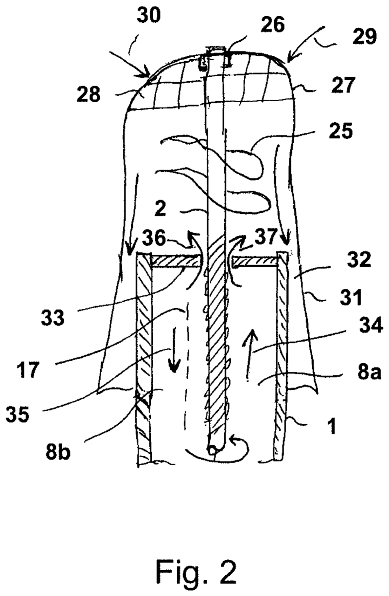

Figur 2 zeigt als Beispiel für eine Verwendung eines Hohlkatheters mit einer antreibbaren Welle eine implantierbare Blutpumpe 24, die als Rotationspumpe mit einem Rotor 25 mit Förderelementen ausgebildet ist. Der Rotor 25 ist direkt mit der Welle 2 verbunden, die am distalen Ende des Rotors 25 in einem Rotationslager 26 im Gehäuse 27 der Blutpumpe gelagert ist. Die Blutpumpe 24 saugt über Ansaugöffnungen 28 an ihrem distalen Ende Blut in Richtung der Pfeile 29, 30 an und transportiert dieses außen an dem Hohlkatheter 1 vorbei über einen durch einen Abströmschlauch 31 gebildeten Ringkanal 32 in ein nicht dargestelltes Blutgefäß.

Die Welle 2 ist am Ende des Hohlkatheters 1 in einem Durchführungslager 33 gelagert, das einerseits hohe Rotationszahlen zulassen, andererseits möglichst dicht sein soll, um einen Flüssigkeitsaustausch entlang der Welle 2 zu verhindern oder zu begrenzen. Es soll insbesondere verhindert werden, dass Blut aus dem Inneren des Gehäuses 27 der Blutpumpe 24 in den Hohlkatheter 1, d. h. in den Kanal 8, gelangt.

In Figur 2 ist, um die Separierung zwischen dem ersten Kanalbereich 8a des Kanals 8 und dem zweiten Kanalbereich/Rückkanal 8b anzudeuten, eine Trennwand 17 gestrichelt dargestellt. Es wird somit das Einströmen der Flüssigkeit durch den ersten Kanalbereich 8a in Richtung des Pfeils 34 zum distalen Ende des Hohlkatheters 1 und das Rückströmen in Richtung des Pfeils 35 durch den zweiten Bereich 8b des Kanals 8 ermöglicht. Damit kann die rotierende Welle 2 entlang ihrer gesamten Länge mit der Flüssigkeit versorgt werden.

Um das Einströmen von Blut in den Kanal 8 zu verhindern, kann ein Überdruck der Flüssigkeit im Inneren des Hohlkatheters 1, d. h. im Kanal 8, eingestellt werden, der dazu führt, dass mit einer sehr geringen Durchflussrate Flüssigkeit aus dem Kanal 8 in das Gehäuse der Blutpumpe 24 einströmt, wie durch die Pfeile 36, 37 angedeutet ist. Beispielsweise kann hier eine Ausflussrate von wenigen Mikrolitern oder Millilitern pro Tag eingestellt werden, die eine Differenz zwischen der Zulaufrate im ersten Kanalbereich 8a und der Rücklaufrate im Rückkanal 8b darstellt. Diese Differenz ist als Differenz der Förderraten zwischen der ersten Pumpe 10 und der zweiten Pumpe 19 einstellbar und messbar.

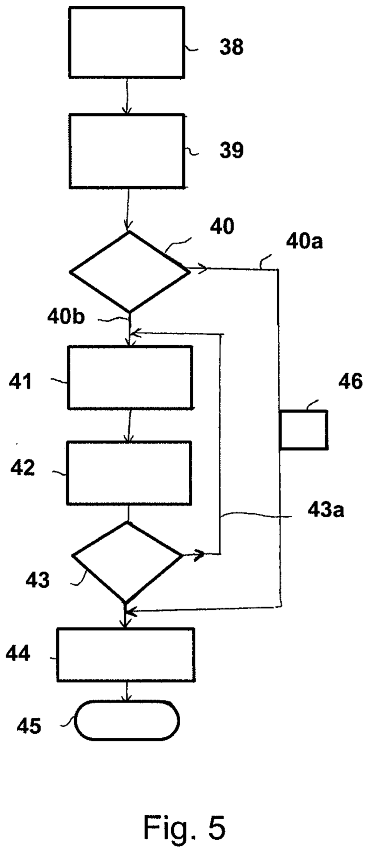

In Figur 5 ist ein Ablaufdiagramm für ein Verfahren zum Betrieb der gezeigten Versorgungseinrichtung dargestellt. In einem ersten Schritt 38 wird eine Entlüftung des Kanals 8 einschließlich des Koppelgehäuses 5 durchgeführt, indem mittels der ersten Pumpe 10 Flüssigkeit zugeführt wird. Nach dem Entlüften des Kanals 8 und der Pumpen, dessen Geschwindigkeit einstellbar sein kann, wird in einem zweiten Schritt 39 festgelegt, in welcher Bewegungsrichtung (Vorlauf/Rücklauf) die Flüssigkeit durch den Kanal 8 bewegt werden soll. Die Membranpumpen 10, 19 sowie die Reservoirs 12, 21 können beide Bewegungsrichtungen der Flüssigkeit zulassen. Abhängig von der Bewegungsrichtung der Flüssigkeit werden die Drücke eingestellt, die durch die Membranpumpen 10, 19 erzeugt werden.

In einem dritten Schritt 40 wird entschieden, ob die Leistungen der Pumpen manuell eingestellt werden sollen. Werden die Pumpen manuell eingestellt, so verläuft der weitere Prozess über den Weg 40a, und in einem Schritt 46 werden die Drücke und/oder Durchflussraten der beiden Pumpen eingestellt. Üblicherweise wird diese Variante wohl gewählt, wenn die Spülrate, d. h. die Durchflussrate durch den Kanal 8, klein und konstant sein soll.

Soll keine manuelle Ansteuerung gewählt werden, so verläuft der weitere Weg über den Pfeil 40b, und es wird in einem vierten Schritt 41 die automatische Ansteuerung der Pumpen begonnen. Hierzu wird zunächst im Schritt 41 der Druck an den zwei Drucksensoren 15, 22 erfasst, hieraus eine Druckdifferenz berechnet und aus dieser in einem fünften Schritt 42 die Ansteuerung der Pumpen 10, 19 durch entsprechende Pulse der Steuereinrichtung 11 berechnet. Dabei kann die angestrebte Druckdifferenz auch zeitlich veränderlich, beispielsweise periodisch variierend sein.

In einem sechsten Schritt 43 wird die erzeugte Druckdifferenz mit der Solldruckdifferenz verglichen. Entspricht die Istdruckdifferenz der Solldruckdifferenz, so wird in einem siebten Schritt 44 beispielsweise die Druckdifferenz oder eine hieraus berechnete Spülrate angezeigt und in einem achten Schritt 45 das Verfahren beendet. Die Beendigung des Verfahrens bedeutet, dass sich die Versorgungseinrichtung in einem stabilen Betriebszustand befindet und die Pumpen 10, 19 entsprechend angesteuert werden und arbeiten. Wird in dem sechsten Schritt 43 festgestellt, dass die Istdruckdifferenz nicht der Solldruckdifferenz entspricht, so springt das Verfahren über den Weg 43a zu dem vierten Schritt 41 zurück, an dem die Druckdifferenz gemessen und hieraus in einem Regelschritt die neue Ansteuerung der Pumpen ermittelt wird.

Anstelle der Druckmessungen und entsprechender Druckregelung des Differenzdrucks kann auch die Durchflussrate gemessen und eine entsprechende Durchflussratendifferenz als Regelgröße eingestellt werden.

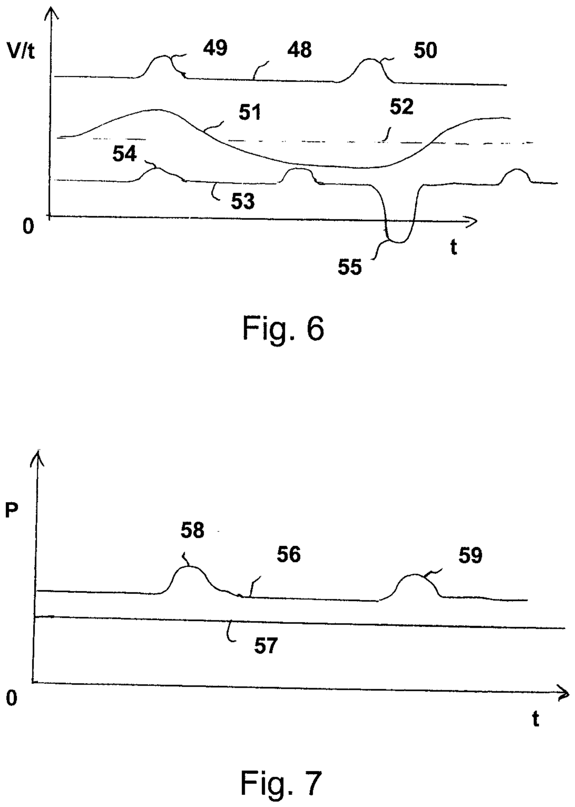

In Figur 6 ist ein typischer zeitlicher Verlauf von Durchflussraten in drei beispielhaften Varianten gezeigt. Auf der y-Achse des Diagramms ist die Durchflussrate in Volumen pro Zeit angegeben, während auf der x-Achse die Zeit aufgetragen ist. Eine erste Kurve 48 zeigt beispielsweise die Durchflussrate, gemessen durch den Sensor 15 oder den Sensor 22, wobei die Durchflussrate über einen großen Teil der Zeit konstant ist, jedoch von Zeit zu Zeit, beispielsweise alle zwanzig Sekunden oder jeweils nach wenigen Minuten, durch eine vorübergehende Anhebung 49, 50 der Durchflussrate verändert wird. Damit wird erreicht, dass sich keine stationäre Strömung in dem Kanal 8 ausbildet, die möglicherweise bestimmte Bereiche des Kanals als sogenannte Totwassergebiete unberührt lässt, so dass dort befindliche Flüssigkeit sich nicht weiterbewegt. Eine Änderung der Durchflussrate erzeugt Wirbel und nichtstationäre Strömungsverhältnisse, die dann auch die Totwassergebiete erfassen und dort die Flüssigkeit austauschen.

Es ist weiterhin Aufgabe einer entsprechenden Steuerung der Durchflussrate, Partikel, die sich in der Flüssigkeit befinden und die beispielsweise durch Abrieb der rotierenden Welle 2 entstehen, möglichst nicht weiterzubewegen, so dass diese nicht durch das in Figur 2 dargestellte Lager 33 im Bereich der Blutpumpe austreten und in das Körperinnere eines Patienten eintreten können.

Wenn man in demselben Diagramm die durch die beiden Sensoren 15, 22 erfassten Durchflussraten aufträgt, so kann sich beispielsweise besonders in den Bereichen 49, 50 eine angehobene Durchflussrate mit einer besonders auffälligen Differenz der Durchflussraten einstellen, was anzeigt, dass in diesen Bereichen 49, 50 stoßweise in sehr kleinen Mengen etwas von der Flüssigkeit aus dem Kanal 8 in das Innere des Pumpengehäuses der Blutpumpe austritt und somit möglicherweise dort abgelagerte Mengen des Blutes aus dem Lager 33 wegspült.

In einer zweiten Variante 51 des Durchflussratenverlaufs wird dieser um einen konstanten Verlauf 52 herum periodisch, beispielsweise in Form einer Sinuskurve, variiert. So ergibt sich ein sich ständig ändernder Durchfluss mit sich ebenfalls ständig ändernden Strömungsverhältnissen, die einen Flüssigkeitsaustausch in allen Bereichen des Kanals 8 garantieren.

In der dritten Variante, die in der Kurve 53 dargestellt ist, wird außer vorübergehenden periodischen Anhebungen 54 der Durchflussrate gelegentlich auch die Durchflussrichtung, gezeigt am Beispiel der Absenkung 55 der Durchflussrate, umgekehrt. Die Umkehrung des Durchflusses bedingt eine Änderung der Flussrichtung der Flüssigkeit im Kanal 8 und damit ebenfalls den Austausch von Flüssigkeit in Totwassergebieten. Eine solche Umkehrung der Flussrichtung kann beispielsweise jeweils im Abstand von fünf bis zehn Minuten geschehen.

In Figur 7 sind Druckmesswerte auf der y-Achse gegen die Zeit t aufgetragen, wobei eine erste Kurve 56 den Druck im Bereich des Sensors 15 und eine zweite Kurve 57 den Druck im Bereich des Sensors 22 anzeigt. Es zeigt sich, dass in zwei Bereichen 58, 59 der Druck durch die erste Membranpumpe 10 vorübergehend angehoben wird, während der Druck im Bereich der Rückleitung, nachgewiesen durch den Sensor 22, konstant bleibt. Dies bedingt, dass in den Bereichen des angehobenen Drucks 58, 59 durch das Lager 33 Flüssigkeit in das Pumpengehäuse abströmt und damit der Druck im Kanal 8 entlastet wird.

Durch die oben beschriebene Erfindung wird eine Versorgungseinrichtung in Form einer Spüleinrichtung für einen Hohlkatheter für eine Blutpumpe realisiert, bei der wenig Verschleißteile eingesetzt werden und somit über lange Zeit ein stabiler Betrieb mit geringen Flüssigkeitsverlusten gewährleistet werden kann.

Der vorliegende Gegenstand umfasst unter anderem die folgenden Aspekte:
1. Versorgungseinrichtung zur Beaufschlagung eines Kanals (8) mit einer Flüssigkeit mit wenigstens einer Membranpumpe (10, 19) sowie mit einer Steuereinrichtung (11), die die Pumpe bezüglich des erzeugten Drucks und/oder der Förderrate ansteuert.
2. Versorgungseinrichtung nach Aspekt 1 mit wenigstens zwei Pumpen (10, 19), insbesondere Membranpumpen, die an voneinander beabstandeten Stellen des Kanals (8) angeordnet sind, sowie mit einer Steuereinrichtung (11), die die Pumpen bezüglich des erzeugten Drucks und/oder der Förderrate einzeln ansteuert.
3. Versorgungseinrichtung nach Aspekt 1 oder 2, dadurch gekennzeichnet, dass jeder Pumpe ein Flüssigkeitsdrucksensor (15, 22) zugeordnet ist.
4. Versorgungseinrichtung nach Aspekt 1, 2 oder 3, dadurch gekennzeichnet, dass jeder Pumpe ein elektrischer Sensor zur Erfassung der Leistungsaufnahme der Pumpe, insbesondere der Stromaufnahme, zugeordnet ist.
5. Versorgungseinrichtung nach Aspekt 1 oder einem der folgenden, dadurch gekennzeichnet, dass jeder Pumpe ein Durchflussratensensor (15, 22) zugeordnet ist.
6. Verfahren zum Betrieb einer Versorgungseinrichtung, die einen Kanal mit einer Flüssigkeit beaufschlagt, mit einer Membranpumpe, dadurch gekennzeichnet, dass die Membranpumpe bezüglich des erzeugten Drucks und/oder der Förderrate angesteuert wird.
7. Verfahren zum Betrieb einer Versorgungseinrichtung, die einen Kanal mit einer Flüssigkeit beaufschlagt, mit zwei an voneinander beabstandeten Stellen des Kanals angeordneten Pumpen (10, 19), dadurch gekennzeichnet, dass die Parameterwerte wenigstens eines Betriebsparameters beider Pumpen (10, 19) aufeinander abgestimmt gesteuert werden.
8. Verfahren nach Aspekt 6 oder 7, dadurch gekennzeichnet, dass an an einer Stelle oder an mehreren verschiedenen, voneinander beabstandeten Stellen in dem Kanal der Flüssigkeitsdruck erfasst wird.
9. Verfahren nach Aspekt 7, dadurch gekennzeichnet, dass die abgestimmten Parameterwerte der beiden Pumpen (10, 19) zeitlich nach einem festen Schema veränderlich sind und insbesondere nach einer Anlaufphase zeitlich periodisch verändert werden.
10. Verfahren nach Aspekt 7, 8 oder 9, dadurch gekennzeichnet, dass die abgestimmten Parameterwerte der beiden Pumpen (10, 19) in einem Verhältnis zueinander stehen, das von erfassten Werten des Flüssigkeitsdrucks im Kanal in vorbestimmter Weise abhängig ist.
11. Verfahren nach Aspekt 7 oder einem der folgenden, dadurch gekennzeichnet, dass die abgestimmten Betriebsparameter beider Pumpen (10, 19) die jeweiligen Förderleistungen sind.
12. Verfahren nach Aspekt 7 oder einem der folgenden, dadurch gekennzeichnet, dass die abgestimmten Betriebsparameter beider Pumpen (10, 19) die jeweiligen Werte des durch die Pumpen erzeugten Flüssigkeitsdrucks sind.
13. Verfahren nach Aspekt 7 oder einem der folgenden, dadurch gekennzeichnet, dass die abgestimmten Betriebsparameter beider Pumpen (10, 19) die jeweiligen Förderleistungen der Pumpen sind.
14. Verfahren nach Aspekt 7 oder einem der folgenden, dadurch gekennzeichnet, dass die abgestimmten Betriebsparameter beider Pumpen (10, 19) die jeweiligen Durchflussraten der Pumpen sind.
15. Verfahren nach Aspekt 7 oder einem der folgenden, dadurch gekennzeichnet, dass zwischen den beiden Pumpen (10, 19) eine feste Druckdifferenz und/oder eine feste Differenz der Durchflussrate eingestellt wird.
16. Verfahren nach Aspekt 15, dadurch gekennzeichnet, dass die Differenz der Durchflussraten beider Pumpen (10, 19) kleiner ist als 100 Milliliter pro Tag, insbesondere kleiner ist als 10 Milliliter pro Tag, insbesondere kleiner als 1 Milliliter pro Tag.
17. Verfahren nach Aspekt 6 oder einem der folgenden, dadurch gekennzeichnet, dass die Pumpe(n) (10, 19) derart angesteuert wird/ werden, dass die Bewegungsrichtung der Flüssigkeit umgekehrt wird.
18. Hohlkatheter mit einer Versorgungseinrichtung nach einem der Aspekte 1 bis 5.
19. Hohlkatheter nach Aspekt 18, enthaltend eine innerhalb des Hohlkatheters drehbare Welle.
20. Hohlkatheter nach Aspekt 18 oder 19, dadurch gekennzeichnet, dass innerhalb des Hohlkatheters eine flexible Welle angeordnet ist.
21. Katheterpumpe, insbesondere Katheterpumpe zum intraventrikulären Betrieb innerhalb eines Herzens, enthaltend einen Hohlkatheter nach einem der Aspekte 18 bis 20.

## Patentansprüche

1. Katheterpumpe zum intraventrikulären Betrieb innerhalb eines Herzens, enthaltend einen Hohlkatheter mit einer Versorgungseinrichtung zur Beaufschlagung eines Kanals (8) des Hohlkatheters mit einer Flüssigkeit, wobei die Versorgungseinrichtung wenigstens eine Membranpumpe (10, 19) sowie eine Steuereinrichtung (11), die die Membranpumpe (10, 19) bezüglich des erzeugten Drucks und/oder der Förderrate ansteuert, aufweist.

2. Katheterpumpe nach Anspruch 1, **dadurch gekennzeichnet, dass** diese als Rotationspumpe ausgeführt ist.

3. Katheterpumpe nach einem der Ansprüche 1 oder 2, **gekennzeichnet durch** einen Rotor (25) mit Förderelementen.

4. Katheterpumpe nach einem der Ansprüche 1 bis 3, **dadurch gekennzeichnet, dass** die Membranpumpe (10, 19) derart ansteuerbar ist, dass die Bewegungsrichtung der Flüssigkeit umgekehrt wird.

5. Katheterpumpe nach Anspruch 4, eingerichtet für eine periodische Umkehrung der Bewegungsrichtung der Flüssigkeit.

6. Katheterpumpe nach einem der Ansprüche 1 bis 5, eingerichtet für eine Transportrichtung der Flüssigkeit von einem proximalen Ende des Hohlkatheters zu einem distalen Ende des Hohlkatheters und durch einen Rückflusskanal zurück in einen Auffangbehälter.

7. Katheterpumpe nach einem der Ansprüche 1 bis 6, **dadurch gekennzeichnet, dass** an einem distalen Ende des Hohlkatheters eine implantierbare Blutpumpe für den Betrieb in einer Herzkammer vorgesehen ist.

8. Katheterpumpe nach einem der Ansprüche 1 bis 7, **gekennzeichnet durch** eine Magneteinrichtung (10a), die als Antrieb der Membranpumpe (10, 19) dient, wobei die Magneteinrichtung (10a) durch die Steuereinrichtung (11) ansteuerbar ist.

9. Katheterpumpe nach einem der vorhergehenden Ansprüche, mit wenigstens zwei Pumpen (10, 19), nämlich Membranpumpen, die an voneinander beabstandeten Stellen des Kanals (8) angeordnet sind, sowie mit einer Steuereinrichtung (11), die die Pumpen bezüglich des erzeugten Drucks und/oder der Förderrate einzeln ansteuert.

10. Katheterpumpe nach Anspruch 9, **dadurch gekennzeichnet, dass** jeder Pumpe ein Flüssigkeitsdrucksensor (15, 22) zugeordnet ist

11. Katheterpumpe nach einem der Ansprüche 9 oder 10, **dadurch gekennzeichnet, dass** jeder Membranpumpe (10, 19) ein elektrischer Sensor zur Erfassung der Leistungsaufnahme der Membranpumpe (10, 19), insbesondere der Stromaufnahme, zugeordnet ist.

12. Kathetherpumpe nach einem der Ansprüche 9 bis 11, **dadurch gekennzeichnet, dass** jeder Membranpumpe (10, 19) ein Durchflussratensensor (15, 22) zugeordnet ist.
